(19) Europäisches Patentamt
European Patent Office
Office européen des brevets

(11) **EP 1 856 986 B1**

(12) **EUROPEAN PATENT SPECIFICATION**

(45) Date of publication and mention
of the grant of the patent:
**18.08.2010 Bulletin 2010/33**

(51) Int Cl.:
*A23L 1/0522* (2006.01)   *A23L 1/09* (2006.01)
*A23L 1/29* (2006.01)   *C08B 31/10* (2006.01)
*C08B 33/02* (2006.01)   *C08B 33/04* (2006.01)
*C08B 35/02* (2006.01)   *C08B 35/04* (2006.01)

(21) Application number: **06076058.4**

(22) Date of filing: **16.05.2006**

(54) **Method for reducing the oil uptake in fried foodstuffs**

Verfahren zur Reduktion der Oelaufnahme in fritierten Lebensmitelln

Méthode pour réduire l'absorption de l'huile dans des produits frits

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(43) Date of publication of application:
**21.11.2007 Bulletin 2007/47**

(73) Proprietor: **Coöperatie Avebe U.A.**
**9641 GK Veendam (NL)**

(72) Inventors:
• **de Vries, Hendrik Jan**
**9551 TW Sellingen (NL)**

• **Buwalda, Pieter Lykle**
**9718 MJ Groningen (NL)**

(74) Representative: **van Loon, C.J.J. et al**
**Vereenigde**
**Johan de Wittlaan 7**
**2517 JR Den Haag (NL)**

(56) References cited:
**EP-A- 1 462 005**   **WO-A-00/42076**
**WO-A-02/067696**   **WO-A-2005/067720**
**US-A- 5 928 700**   **US-B1- 6 558 730**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a method for reducing the oil uptake of fried, or extruded and fried food products.

Obesity, a condition resulting from excessive fat storage in the body, is becoming an important issue in modern society. Obesity is a major public health concern because it predisposes the individual to many disorders, such as non-insulin dependent diabetes, hypertension, stroke, and coronary artery disease. It has also been associated with an increased incidence of certain cancers, notably cancers of the colon, rectum, prostate, breast, uterus, and cervix. Among the causes of obesity is the consumption of high-calorie food, such as food with a high fat content. Well-known examples of such foods are deep-fried snack products.

In order to lower the consumption of fat, a wide range of low-fat food diets have been designed. These diets mostly involve a dramatic change in consumption habits. It is often observed that people abandon the diet because there is a considerable change in taste compared to the usually consumed food. Further, a lot of people tend to drop back into their old consumption habits after a successful diet.

It is desirable to manufacture known food-products that have a high consumer acceptability with a lower amount of fat. A wide range of such diet food products is already known. However, it is still a challenge to provide a fried food product with a reduced fat content.

An object of the present invention is to provide a method for reducing the tendency of a food product to take up oil when being fried.

Surprisingly, it has been found that this object can be met by the method of the invention for reducing the oil uptake of a food product during frying, comprising partially replacing said cook-up starch and/or instant starch in the ingredients of said food product by, or adding a hydrophobic starch. Preferably, the food product is a cook-up starch and/or instant starch containing food product.

In the context of this invention "partially replacing" is meant to be the partial removal of an original ingredient from the recipe and addition of another ingredient in the recipe. It is not necessary that the added ingredient has the exact same weight as the partially removed ingredient. Preferably, the weight of the added component is in the range of 80-120 % of the weight of the partially removed component, more preferably 90-110 %.

According to the invention, it is not necessary to partially remove a cook-up starch and/or instant starch from the recipe. Mere addition of the hydrophobic starch also leads to the desired effect. Preferably, not more than 15 wt.% of hydrophobic starch is added with respect to the total weight of components in the original recipe, more preferably not more than 10 wt.%.

It has been found that the resulting food products have a remarkable lower tendency to take up oil when being fried than conventional food products. The uptake of oil in comparison to conventional food products can be reduced up to 30 %.

The starch on which the hydrophobic starch according to the invention is based can, in principle, be derived from any botanical source. Both root or tuber starches, such as cassava or potato starch, and cereal and fruit starches, such as maize, rice, wheat or barley starches can be used. Legume starches, such as pea or bean starches, can also be used.

Natural starches typically have a more or less fixed ratio of the two components of starch, amylose and amylopectin. Of some starches, such as maize or rice starch, a natural occurring variety exists which contains essentially only amylopectin. These starches, which are normally called waxy starches, can also be used. Of other starches, such as potato or cassava starch, there are genetically modified or mutant varieties which also essentially only contain amylopectin. It will be understood that the use of these varieties, typically comprising more than 80 wt.%, preferably more than 95 wt.%, based on dry weight of the starch, of amylopectin, is also within the scope of the invention. Finally, also starch varieties which are high in amylose, such as high amylose potato starch, can be used for the preparation of an emulsifier according to the invention. In accordance with the invention, starches of all amylose to amylopectin ratios may be used.

In order to obtain the hydrophobic starch of the invention, a hydrophobic substituent, comprising an alkyl or alkenyl chain having 4-24 carbon atoms, is attached to a starch by an ether, ester or amide linkage.

When the hydrophobic group is attached to the starch via an ether linkage, the hydrophobic reagent preferably comprises a halide, halohydrin, epoxide or glycidyl group as reactive site. The alkyl or alkenyl chain of the agent can vary from 4-24 carbon atoms, preferably from 7-20 carbon atoms. Suitable examples of hydrophobic reagents to provide an ether linkage are cetyl bromide, lauryl bromide, butylene oxide, epoxidised soybean fatty alcohols, epoxydized linseed fatty alcohols, allyl glycidyl ether, propyl glycidyl ether, butyl glycidyl ether, decane glycidyl ether, lauryl glycidyl ether, lauryl phenyl glycidyl ether, myristoyl glycidyl ether, cetyl glycidyl ether, palmityl glycidyl ether, stearyl glycidyl ether, linolyl glycidyl ether and mixtures thereof. Other etherification agents which may be used to react with starch are alkyl halides containing at least four carbon atoms, such as 1-bromodecane, 10-bromo-1-decanol, and 1-bromododecane.

It is also possible that a charged hydrophobic group is introduced. A hydrophobic cationic group can be attached via an ether linkage by reaction of the starch with a reagent comprising a quaternary ammonium group, for example a 1-chloro-2-hydroxypropyltrialkyl ammonium salt or a glycidyltrialkyl ammonium salt. The alkyl chains of this quaternary ammonium group can vary from 1-24 carbon atoms, preferably from 7-20 carbon atoms, wherein at least one of the alkyl chains of the quaternary ammonium group comprises 4-24 carbon atoms. Preferably, the other alkyl chains have less than 7 carbon atoms. For example 1-chloro-2-hydroxypropyldimethyllauryl ammonium salt, 1-chloro-2-hydroxypropyldimethylmyristoyl ammonium salt, 1-chloro-2-hydroxypropyldimethylcetyl, 1-chloro-2-hydroxypropyldimethylstearyl, glycidyldimethyllauryl ammonium salt, glycidyldimethylmyristoyl ammonium salt, glycidyldimethylcetyl ammonium salt, glycidyldimethylstearyl ammonium salt, dialkylaminoethyl halide, or mixtures of the above can be applied as hydrophobic cationisation reagent. A hydrophobic cationic group may be introduced by reaction with tertiary ammonium groups such as chloroethyldialkylamine hydrogen chloride salt. The alkyl chain of this tertiary ammonium group may vary from 1 to 24 carbon atoms. The reaction for introducing the hydrophobic cationic group may be performed analogous to the procedure disclosed in EP-A-0 189 935. A hydrophobic anionic group can be attached applying a 2-chloro-aminodialkyl acid as reagent, for instance analogous to the procedure disclosed in EP-A-0 689 829.

When the hydrophobic group is attached to the starch via an ester linkage, several kinds of reagents, such as alkyl anhydrides can be applied. The alkyl chain can vary from 4-24 carbons, preferably from 7-20 carbons. Especially, mixed anhydrides as octanoic acetic anhydride, decanoic acetic anhydride, lauroyl acetic anhydride, myristoyl acetic anhydride are suitable alkyl anhydrides.

Further, hydrophobic anionic groups may be attached to the amylopectin starch. This may be accomplished by reaction of the specific starch with an alkyl succinic anhydride or alkenyl succinic anhydride. The alkyl chain can vary from 4-24 carbons, preferably from 7-20 carbons. Octenyl succinic anhydride, nonyl succinic anhydride, decyl succinic anhydride, dodecenyl succinic anhydride are most commonly applied. The esterification reaction to introduce the desired alkyl or alkenyl succinate groups can be performed in any known manner, for instance analogous to the procedure disclosed in US-A-5 776 476. Preferably, the starch is reacted with an alkyl or alkenyl succinic anhydride comprising an alkyl or alkenyl group having from 8-12 carbon atoms. Octenyl succinic anhydride, nonyl succinic anhydride, decyl succinic anhydride, dodecenyl succinic anhydride are particularly preferred, while the highest preference is for octenyl succinic anhydride. Accordingly, the alkyl or alkenyl succinated starch preferably is octenyl succinated, nonyl succinated, decyl succinated, or dodecenyl succinated starch, even more preferably octenyl succinated starch.

For the preparation of a hydrophobic group linked to carboxymethyl amylopectin starch by an amide group the procedure as described in WO-A-94/24169 can analogously be applied. Examples of suitable reagents for introduction of an amide group include fatty amines comprising saturated or unsaturated hydrocarbon groups having from 4 to 30 carbon atoms. Branched hydrocarbon groups are not excluded, but linear chains are preferred. Preferably, the fatty radical originates from a $C_{12}$ to $C_{24}$ fatty amine. Particularly favorable results are obtained if the fatty amine is selected from the group consisting of n-dodecylamine, n-hexadecylamine, n-octadecylamine, cocoamine, tallowamine, hydrogenated N-tallow-1,3-diaminopropane, N-hydrogenated tallow-1,3-diaminopropane, and N-oleyl-1,3-diaminopropane. Such fatty amines are known under the trade names Armeen and Duomeen (AKZO Chemicals).

The degree of hydrophobic substitution, *i.e.* DS, defined as the average number of moles of hydrophobic substituents per mole glucose units, achieved in a process according to the invention, may vary particularly depending upon the envisaged application of the product. Generally, the DS will be greater than zero, preferably from 0.005 to about 0.5, more preferably from 0.01 to 0.1. It is surprising to note that even a very small DS leads to a relatively large effect.

The reaction with the hydrophobic reagent can be carried out in a suspension of the starch, i.e. using starch which is not pre-gelatinised, in a suspension of the starch, or under semi-dry conditions.

Preferably, water is used as a solvent when the reaction is performed in suspension or solution. When the used hydrophobic reagent has a low solubility in water, combinations of water and suitable water mixable organic solvents may be employed. Suitable organic solvents include, but are not limited to, methanol, ethanol, i-propanol, n-propanol, t-butanol, sec-butanol, methylethylketon, tetrahydrofuran, dioxan, and acetone. The reaction in solution is preferably performed using a reaction mixture comprising more than 20 wt.% of the starch and less than 80 wt.% of the solvent. More preferably, the starch content in the reaction mixture lies between 20 and 40 wt.%, whereas the solvent content preferably lies between 80 and 60 wt.%. If desired, the solution may be concentrated and/or purified, *e.g.* using dialysis, ultrafiltration, ultracentrifugation, or the like. An autoclave in combination with a dryer (drum dryer; spray dryer) may be used as a reaction vessel. The reaction is further performed under conditions which are well-known for analogous reactions. The pH lies preferably between 7 and 13. Preferably, the reaction is carried out in the presence of a caustic catalyst, such as an alkali metal hydroxide or the like.

It is possible to chemically modify the hydrophobic starch of the invention by acid degradation and dextrinisation.

Modification of the hydrophobic starch can also be achieved by using an enzyme such as glycosyl transferase (E.C. 2.4). Other enzymes that may be used for enzymatic modification include α-amylase, β-amylase, gluco-amylase, iso-amylase, pullulanase, CGT-ase and branching enzymes.

Preferably, at most 15 wt.% and more preferably at most 10 wt.% of the cook-up starch and/or instant starch is replaced with hydrophobic starch. The hydrophobic starch hardly affects the sensoric properties of the food product. However, an amount of hydrophobic starch in the food product of more than 15 wt.% may have a negative effect on the shape and smoothness of the food product. In order to have a significant effect on the oil uptake of the food product the amount of hydrophobic starch in the food product is 1-15 wt.%, preferably at least 2-10 wt.%, more preferably 3-5 wt.%.

Instead of the instant version, also a cook-up version of the hydrophobic starch may be used. Cook-up starch is normally applied in larger amounts in a food product recipe. Replacing part of the cook-up starch will accordingly have less influence on the resulting product than replacing an equal amount of the instant starch. Moreover, cook-up starch is cheaper than instant starch.

The food product recipe may also comprises an emulsifier. Although the oil absorption of the food product is consequently higher, the effect of the method according to the invention is remarkable. Often fats are used as emulsifiers. Typical emulsifiers that are used in food products include but are not limited to monoglycerides, such as Myvatex Mighty Soft, and triglycerides.

The food product recipe can further comprise other ingredients which are exemplified but not limited to salt, potato granules, potato flakes, seasoning, colouring agents.

According to the method of the invention either cook-up starch or instant starch, or both cook-up and instant starch may be replaced by the hydrophobic starch. It is also possible to (partially) replace other ingredients such as potato flakes, potato granules, or other starches such as octenyl succinated starches. The total starch content of the food product preferably is the same as the total starch content of the original food product which does not contain the hydrophobic starch.

For the preparation of the food product the different ingredients of the food product recipe are normally mixed. This mixing can for example be carried out in a blender. Subsequently, the mixed ingredients may be extruded to form the food products to be fried. The extrusion process is not only performed to form the product but also to "cook" the starch.

In a further aspect, the invention is directed to a food product which is obtainable by the method of the invention. Such food products are typically snack food products, such as potato chips. These food products take up less fat during frying than the usual food products.

In another aspect, the invention is directed to the use of the hydrophobic starch as described hereinabove as an oil uptake reducing agent.

The invention is also directed to the use of the hydrophobic starch as described hereinabove in the manufacture of a medicament for treating obesity.

The invention will now be illustrated by the following examples, which should not be interpreted as limiting the invention in any way.

**Examples**

Determination of the degree of substitution

The degree of substitution (DS) is defined as the amount of ester in mol per mol of glucose units. The degree of substitution of is measure according to the following procedure.

Approximately 10 g (dry substance) product and 10 mL methanol are mixed. The mixture is poured into 50 mL water. The suspension is stirred and some drops of phenolphthalein solution in 50 wt.% ethanol are added.

The suspension is titrated with a 0.1 N aqueous NaOH solution to light red. Then 25.00 mL 0.1 N aqueous NaOH solution is added. The mixture is stirred at 40 °C during 24 hours and then the mixture is cooled to room temperature and titrated to colourless with 0.1 N aqueous HCl solution. Beside, the ester substituted products also a non-substituted product is measured as control. The DS is calculated with the formula:

$$DS = \frac{162 + 0.1 \times (25.00 - A)}{B - (Y + 0.1 \times (25.00 - A))}$$

wherein

A = mL 0.1 N HCl of the product - mL 0.1 N HCl of the control;

B = mg product (dry substance); and

Y = 210 if substituent is octenylsuccinic ester or

Y = 266 if substituent is dodecenylsuccinic ester or tetrapropenylsuccinic anhydride.

Example 1

Mixes were prepared and extruded on a Berstorff ZE-25 twin-screw extruder according to the extrusion recipes of Table 1. Paselli P is drum dried cross-linked potato starch. Paselli MC150 is drum dried OSA-starch. The process conditions of the extruder are also shown in Table 1. After extrusion, the pellets were dried to a moisture content of 9.5-10 wt.% and fried for 10 seconds at 190 °C. The oil content of the fried pellets was determined by refluxing a known amount of grinded pellets using petroleum ether. The oil content is calculated based on the gravimetric principle. The results are given in Table 1. The results show a significant decrease of the oil uptake when cook-up starch is replaced with octenyl succinic anhydride modified starch. For example, the reduction of an oil content of 224 mg/g of sample 1 to an oil content of 160 mg corresponds to a reduction of 28.6 %.

Table 1

| sample | | 1 | 2 | 3 | 4 |
|---|---|---|---|---|---|
| potato granules Rixona (wt.%) | | 40 | 40 | 40 | 40 |
| cook-up potato starch (wt.%) | | 53 | 52.5 | 51 | 48 |
| Paselli P (wt.%) | | 6 | 6 | 6 | 6 |
| Paselli MC150 (wt.%) | | 0 | 0.5 | 2 | 5 |
| salt (wt.%) | | 1 | 1 | 1 | 1 |
| moisture content dry mix (wt.%) | | 14.3 | 14.3 | 14.3 | 14.3 |
| oil content of fried pellet (mg per g of total product) | | 224 | 208 | 186 | 160 |
| process conditions | set | | | | |
| Temperature zone 1 in °C | | | | | |
| Temperature zone 2 in °C | 50 | 50 | 50 | 50 | 50 |
| Temperature zone 3 in °C | 50 | 50 | 50 | 50 | 50 |
| Temperature zone 4 in °C | 50 | 50 | 50 | 50 | 50 |
| Temperature zone 5 in °C | 50 | 50 | 50 | 50 | 50 |
| Temperature zone 6 in °C | 60 | 60 | 60 | 60 | 60 |
| Temperature zone 7 in °C | 60 | 61 | 60 | 60 | 59 |
| Temperature zone 8 in °C | 65 | 65 | 65 | 65 | 65 |
| Temperature mass in °C | 76 | 76 | 76 | 76 | 78 |
| screw speed in rpm | 200 | 200 | 200 | 200 | 200 |
| energy consumption in kW/h | | 2.4 | 2.4 | 2.4 | 2.4 |
| Torque in % | | 60 | 60 | 60 | 63 |
| product flow in kg/h | | 5.4 | 5.4 | 5.4 | 5.3 |
| product flow (set) | 2 | 2 | 2 | 2 | 2 |
| water flow (set) | 40 | 40 | 40 | 40 | 40 |
| die configuration | 0.7× 15 mm sleeve | | | | |
| screw configuration (long barrel) | 6x transport E37.5; 1× mixing element 37.5; 9× transport E37.5; 1× ring 2.5; 2× blister 24.5; 1× ring 2.5; 15× transport E25; tip | | | | |

### Example 2

For the preparation of samples 5-8 the screw configuration of the extruder was changed and 1 % of emulsifier (Myvatex Mighty Soft) was added to the food product recipes. The exact recipes and process conditions are given in Table 2. The dried pellets were fried again and the oil content of the fried pellets was determined. The results in Table 2 show the decrease of oil content with increasing octenyl succinic anhydride modified starch content.

Table 2

| sample | 5 | 6 | 7 | 8 |
|---|---|---|---|---|
| potato granules Rixona (wt.%) | 40 | 40 | 40 | 40 |
| cook-up potato starch (wt.%) | 52 | 51 | 49 | 47 |
| Paselli P (wt.%) | 6 | 6 | 6 | 6 |
| Paselli MC150 (wt.%) | 0 | 1 | 3 | 5 |

(continued)

| sample | | 5 | 6 | 7 | 8 |
|---|---|---|---|---|---|
| salt (wt.%) | | 1 | 1 | 1 | 1 |
| emulsifier (Myvatex Mighty Soft) | | 1 | 1 | 1 | 1 |
| moisture content dry mix (wt.%) | | 14.1 | 14.2 | 14.4 | 14.6 |
| oil content of fried pellet (mg per g of total product) | | 231 | 215 | 204 | 192 |
| **process conditions** | **set** | | | | |
| Temperature zone 1 in °C | | 37 | 39 | 39 | 38 |
| Temperature zone 2 in °C | 55 | 54 | 55 | 55 | 55 |
| Temperature zone 3 in °C | 60 | 60 | 60 | 60 | 60 |
| Temperature zone 4 in °C | 60 | 62 | 63 | 62 | 63 |
| Temperature head in °C | 65 | 74 | 70 | 70 | 70 |
| Temperature mass in °C | 76 | 80 | 83 | 83 | 83 |
| screw speed in rpm | 160 | 160 | 160 | 160 | 160 |
| energy consumption in kW/h | | 1.1 | 1.8 | 1.8 | 1.8 |
| Torque in % | | 40 | 55 | 55 | 55 |
| product flow in kg/h | | 5.3 | 6.7 | 7 | 6.6 |
| product flow (set) | 2 | 2 | 2.6 | 2.6 | 2.6 |
| water flow (set) | 40 | 45 | 40 | 40 | 40 |
| die configuration | | 0.7×15 mm sleeve | | | |
| screw configuration (short barrel) | | 6x transport E37.5; 1× mixing element 37.5; 4× E37.5; 2× E25; 1× ZB37.5; 6× E25; 2× blister; 1× ring; 1× ZB15; 3× E25; tip | | | |

## Example 3

Subsequently, samples 9-11 were prepared in which HV041221 (a cook-up potato OSA-starch) in amounts varying from 1-5 wt.% was used. The details are given in Table 3. The results of the determination of the oil content in the fried pellets demonstrate the same functionality of the cook-up octenyl succinic anhydride modified starch. The oil absorption is reduced with almost 22% when 5% cook-up octenyl succinic anhydride modified starch is added to the food product recipe.

Table 3

| sample | | 9 | 10 | 11 |
|---|---|---|---|---|
| potato granules Rixona (wt.%) | | 40 | 40 | 40 |
| cook-up potato starch (wt.%) | | 52 | 50 | 47 |
| Paselli P (wt.%) | | 6 | 6 | 6 |
| HV041221 (wt.%) | | 1 | 2 | 5 |
| salt (wt.%) | | 1 | 2 | 2 |
| moisture content dry mix (wt.%) | | 14.6 | 14.5 | 14.3 |
| oil content of fried pellet (mg per g of total product) | | 221 | 202 | 175 |
| **process conditions** | **set** | | | |
| Temperature zone 1 in °C | | 38 | 35 | 34 |
| Temperature zone 2 in °C | 50 | 56 | 50 | 50 |
| Temperature zone 3 in °C | 55 | 61 | 55 | 55 |
| Temperature zone 4 in °C | 60 | 62 | 70 | 70 |
| Temperature head in °C | 65 | 65 | 66 | 65 |
| Temperature mass in °C | 76 | 78 | 77 | 77 |

(continued)

| process conditions | set | | | |
|---|---|---|---|---|
| screw speed in rpm | 160 | 160 | 160 | 160 |
| energy consumption in kW/h | | 1.6 | 1.2 | 1.3 |
| Torque in % | | 48 | 42 | 43 |
| product flow in kg/h | | 7 | 5 | 5.2 |
| product flow (set) | 2 | 2.6 | 2 | 2 |
| water flow (set) | 40 | 40 | 40 | 40 |
| die configuration | slit 0.7×15 mm | | | |
| screw configuration | 4× E37.5; 2× E25; 1× ZB37.5; 6x E25; 2× blister; 1× ring; 1× ZB15; 3× E25; tip | | | |

Example 4

This example demonstrates that the addition of a hydrophobic starch is needed in order to reduce the oil uptake, and that other starch derivatives do not possess this functionality. The exact recipe and process conditions are given in Table 4. SnackTex C100 is drum dried amylopectin potato starch. Perfectamyl AC is acetyl cook-up potato starch. Farinex VA 15 is cross-linked hydroxy propyl potato starch The results show a significant lower oil uptake when 2 % of octenyl succinic anhydride modified starch is used in the recipe.

Table 4

| sample | | 12 | 13 | 14 | 15 | 16 | 17 |
|---|---|---|---|---|---|---|---|
| potato granules Rixona (wt.%) | | 40 | 40 | 40 | 40 | 40 | 40 |
| cook-up potato starch (wt.%) | | 50 | 50 | 50 | 50 | 50 | 50 |
| Paselli P (wt.%) | | 8 | 6 | 6 | 6 | 6 | 6 |
| SnackTex C100 (wt.%) | | 0 | 2 | 0 | 0 | 0 | 0 |
| Paselli MC150 (wt.%) | | 0 | 0 | 2 | 0 | 0 | 0 |
| HV041221 (wt.%) | | 0 | 0 | 0 | 2 | 0 | 0 |
| Perfectamyl AC (wt.%) | | 0 | 0 | 0 | 0 | 2 | 0 |
| Farinex VA 15 (wt.%) | | 0 | 0 | 0 | 0 | 0 | 2 |
| salt (wt.%) | | 2 | 2 | 2 | 2 | 2 | 2 |
| moisture content dry mix (wt.%) | | 14.2 | 14.2 | 14.1 | 14.1 | 14.3 | 14.3 |
| oil content of fried pellet (mg per g of total product) | | 218 | 223 | 180 | 185 | 216 | 219 |
| process conditions | set | | | | | | |
| Temperature zone 1 in °C | | 35 | 34 | 34 | 35 | 34 | 34 |
| Temperature zone 2 in °C | 50 | 50 | 50 | 51 | 51 | 50 | 50 |
| Temperature zone 3 in °C | 55 | 55 | 55 | 55 | 55 | 55 | 55 |
| Temperature zone 4 in °C | 60 | 70 | 70 | 70 | 70 | 70 | 70 |
| Temperature head in °C | 65 | 66 | 66 | 67 | 66 | 66 | 66 |
| Temperature mass in °C | 76 | 77 | 79 | 80 | 78 | 80 | 79 |
| screw speed in rpm | 160 | 160 | 160 | 160 | 160 | 160 | 160 |
| energy consumption in kW/h | | 1.4 | 1.4 | 1.3 | 1.3 | 1.2 | 1.2 |
| Torque in % | | 50 | 51 | 51 | 52 | 53 | 52 |
| product flow in kg/h | | 5.3 | 5.3 | 5.4 | 5.4 | 5.3 | 5.2 |
| product flow (set) | 2 | 2 | 2 | 2 | 2 | 2 | 2 |
| water flow (set) | 40 | 40 | 40 | 40 | 40 | 40 | 40 |
| water supply kg/h | 1.6 | 1.6 | 1.6 | 1.6 | 1.6 | 1.6 | 1.6 |
| die configuration | slit 0.7×15 mm | | | | | | |
| screw configuration | 4× E37.5; 2× E25; 1× ZB37.5; 6× E25 | | | | | | |

The sensoric evaluation of the different samples is shown in Table 5. It can be concluded that the hydrophobic starch hardly influences the sensoric properties of the food product.

Table 5

| sample | Example 1 | | | | Example 2 | | | | Example 3 | | | Example 4 | | | | | |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 |
| visual expansion (small-large: 0-5) | 2.5 | 2.5 | 2 | 2 | 2.5 | 2.5 | 3 | 2.5 | 2 | 2.5 | 2.5 | 2.5 | 2.5 | 2.5 | 2.5 | 3 | 2.5 |
| smoothness (not-very: 0-5) | 2.5 | 2.5 | 2.5 | 2 | 2.5 | 2.5 | 2.5 | 2 | 3 | 2.5 | 2.5 | 2.5 | 3 | 2.5 | 3 | 2.5 | 2.5 |
| bite (hard-soft: 0-5) | 2.5 | 3 | 3 | 3 | 2.5 | 3 | 3 | 3 | 3 | 3 | 3 | 2.5 | 2.5 | 2.5 | 2.5 | 2.5 | 2.5 |
| crispiness (not-very: 0-5) | 3 | 2.5 | 2.5 | 2 | 2 | 3 | 3 | 2.5 | 3 | 2 | 2 | 3 | 2.5 | 2.5 | 3 | 3 | 3 |
| shape snacks (irregular-regular 0-5) | 2.5 | 2.5 | 2.5 | 2 | 2.5 | 3 | 2.5 | 2 | 3 | 2 | 2.5 | 2.5 | 2 | 2.5 | 2 | 2.5 | 2.5 |

**Claims**

1.  A method for reducing the oil uptake of a food product during frying, comprising partially replacing of cook-up starch and/or instant starch in the ingredients of said food product by or adding a hydrophobic starch, which hydrophobic starch is obtained by reacting starch with a hydrophobic agent comprising an alkyl or alkenyl chain having 4-24 carbon atoms.

2.  A method according to claim 1, wherein food product is a cook-up starch and/or instant starch containing food product.

3.  A method according to claim 1 or 2, wherein the hydrophobic starch is an alkyl or alkenyl succinated starch.

4.  A method according to claim 3, wherein the alkyl or alkenyl succinated starch is octenyl succinated, nonyl succinated, decyl succinated, or dodecenyl succinated starch.

5.  A method according to claim 4, wherein the starch is an octenyl succinated starch.

6.  A method according to any of the claims 3-5, wherein the degree of substitution (DS) of the alkyl or alkenyl succinated starch is from 0.005 to 0.5, preferably from 0.01 to 0.1.

7.  A method according to any of the preceding claims, wherein the hydrophobic starch is a potato, cassava, wheat, barley, maize, rice, pea, or bean starch.

8.  A method according to any of the preceding claims, wherein the hydrophobic starch is chemically modified by acid degradation or dextrinisation.

9.  A method according to any of the preceding claims, wherein the hydrophobic starch is enzymatically modified.

10. A method according to claim 9, wherein the enzymatic modification is the result of an enzyme chosen from the group consisting of glycosyl transferase, α-amylase, β-amylase, gluco-amylase, iso-amylase, pullullanase, CGT-ase and branching enzymes.

11. A method according to any of the preceding claims, wherein the hydrophobic starch has an amylose content of from 20-70 wt.%, preferably 30-50 wt.%, based on dry weight of the starch.

12. A method according to any of the preceding claims, wherein the hydrophobic starch has an amylopectin content higher than 80 wt.%, preferably higher than 95 wt.%, based on dry weight of the starch.

13. A method according to any of the preceding claims, wherein the hydrophobic starch is a cook-up hydrophobic starch.

14. A method according to any of the preceding claims, wherein said food product comprises an emulsifier.

15. A method according to any of the claims 2-14, wherein at most 15 wt.% of said cook-up starch and/or instant starch is replaced by said hydrophobic starch, preferably at most 10 wt.%, more preferably at most 5 wt.%.

16. A method according to any of the claims 2-15, wherein said cook-up starch is a potato, cassava, wheat, barley, maize, rice, pea, or bean starch.

17. Use of a hydrophobic starch as an oil uptake reducing agent during frying, wherein the hydrophobic starch is obtained by reacting starch with a hydrophobic agent comprising an alkyl or alkenyl chain having 4-24 carbon atoms.

**Patentansprüche**

1. Verfahren zum Reduzieren der Ölaufnahme eines Lebensmittelprodukts während des Frittierens, bei dem Kochstärke und/oder Instantstärke in den Zutaten des Lebensmittelprodukts teilweise durch hydrophobe Stärke ersetzt werden/wird oder hydrophobe Stärke zugegeben wird, wobei die hydrophobe Stärke durch Umsetzen von Stärke mit hydrophobem Mittel mit einer Alkyl- oder Alkenylkette mit 4 bis 24 Kohlenstoffatomen erhalten ist.

2. Verfahren nach Anspruch 1, bei dem das Lebensmittelprodukt ein Kochstärke- und/oder Instantstärke-haltiges Lebensmittelprodukt ist.

3. Verfahren nach Anspruch 1 oder 2, bei dem die hydrophobe Stärke Alkyl- oder Alkenyl-succinierte Stärke ist.

4. Verfahren nach Anspruch 3, bei dem die Alkyl- oder Alkenyl-succinierte Stärke Octenyl-succinierte, Nonylsuccinierte, Decyl-succinierte oder Dodecyl-succinierte Stärke ist.

5. Verfahren nach Anspruch 4, bei dem die Stärke Octenyl-succinierte Stärke ist.

6. Verfahren nach einem der Ansprüche 3 bis 5, bei dem der Substitutionsgrad (SG) der Alkyl- oder Alkenyl-succinierten Stärke 0,005 bis 0,5 beträgt, vorzugsweise 0,01 bis 0,1.

7. Verfahren nach einem der vorhergehenden Ansprüche, bei dem die hydrophobe Stärke Kartoffel-, Maniok-, Weizen-, Gersten-, Mais-, Reis-, Erbsen- oder Bohnenstärke ist.

8. Verfahren nach einem der vorhergehenden Ansprüche, bei dem die hydrophobe Stärke durch Säureabbau oder Dextrinierung chemisch modifiziert ist.

9. Verfahren nach einem der vorhergehenden Ansprüche, bei dem die hydrophobe Stärke enzymatisch modifiziert ist.

10. Verfahren nach Anspruch 9, bei dem die enzymatische Modifizierung das Ergebnis eines Enzyms ist, das ausgewählt ist aus der Gruppe bestehend aus Glycosyltransferase, $\alpha$-Amylase, $\beta$-Amylase, Glucoamylase, Isoamylase, Pullulanase, CGTase und Verzweigungsenzymen.

11. Verfahren nach einem der vorhergehenden Ansprüche, bei dem die hydrophobe Stärke einen Amylosegehalt von 20 bis 70 Gew.-% besitzt, vorzugsweise 30 bis 50 Ges.-%, bezogen auf das Trockengewicht der Starke.

12. Verfahren nach einem der vorhergehenden Ansprüche, bei dem die hydrophobe Stärke einen Amylopektingehalt von höher als 80 Gew.-% besitzt, vorzugsweise höher als 95 Ges.-%, bezogen auf das Trockengewicht der Stärke.

**13.** Verfahren nach einem der vorhergehenden Ansprüche, bei dem die hydrophobe Stärke hydrophobe Kochstärke ist.

**14.** Verfahren nach einem der vorhergehenden Ansprüche, bei dem das Lebensmittelprodukt Emulgator enthält.

**15.** Verfahren nach einem der Ansprüche 2 bis 14, bei dem höchstens 15 Gew.-% der Kochstärke und/oder Instantstärke durch die hydrophobe Stärke ersetzt werden, vorzugsweise höchstens 10 Gew.-%, bevorzugter höchstens 5 Ges.-%.

**16.** Verfahren nach einem der Ansprüche 2 bis 15, bei dem die Kochstärke Kartoffel-, Maniok-, Weizen-, Gersten-, Mais-, Reis-, Erbsen- oder Bohnenstärke ist.

**17.** Verwendung von hydrophober Stärke als ein die Ölaufnahme reduzierendes Mittel während des Frittierens, wobei die hydrophobe Stärke durch Umsetzen von Stärke mit einem hydrophoben Mittel mit einer Alkyl- oder Alkenylkette mit 4 bis 24 Kohlenstoffatomen erhalten ist.

**Revendications**

**1.** procédé de réduction d'absorption d'huile d'un aliment alimentaire pendant la friture, comprenant le replacement partiel d'amidon cuit et/ou d'amidon prégélatinisé dans les ingrédients dudit produit alimentaire par ou en ajoutant un amidon hydrophobe, lequel amidon hydrophobe est obtenu en faisant réagir un amidon avec un agent hydrophobe comprenant une chaîne alkyle ou alcényle constituée de 4 à 24 atomes de carbone.

**2.** Procédé selon la revendication 1, dans lequel le produit alimentaire est un produit alimentaire contenant un amidon cuit et/ou un amidon prégélatinisé.

**3.** Procédé selon la revendication 1 ou 2, dans lequel l'amidon hydrophobe est un amidon à base de succinate d'alkyle ou d'alcényle.

**4.** Procédé selon la revendication 3, dans lequel l'amidon à base de succinate d'alkyle ou d'alcényle est un amidon de succinate d'octényle, de succinate de nonyle, de succinate de décyle ou de succinate de dodécényle.

**5.** Procédé selon la revendication 4, dans lequel l'amidon est un amidon à base de succinate d'octényle.

**6.** Procédé selon l'une quelconque des revendications 3 à 5, dans lequel le degré de substitution (DS) de l'amidon à base de succinate d'alkyle ou d'alcényle est compris dans la plage allant de 0,005 à 0,5, de préférence de 0,01 à 0,1.

**7.** Procédé Selon l'une quelconque des revendications précédentes, dans lequel l'amidon hydrophobe est un amidon de pomme de terre, de manioc, de blé, d'orge, de mais, de riz, de poids ou de haricot.

**8.** Procédé selon l'une quelconque des revendications précédentes, dans lequel l'amidon hydrophobe est chimiquement Modifié par dégradation ou dextrinisation acide.

**9.** procédé selon l'une quelconque des revendications précédentes, dans lequel l'amidon hydrophobe est enzymatiquement modifié.

**10.** Procédé selon la revendication 9, dans lequel la modification enzymatique est due à une enzyme choisie dans le groupe constitué par la glycosyl-transférase, l'α-amylase, la β-amylase, la gluco-amylase, l'isoamylase, la pullullanase, la CGT-ase et les enzymes de ramification.

**11.** Procédé selon l'une quelconque des revendications précédentes, dans lequel l'amidon hydrophobe possède une teneur en amylose comprise dans la plage allant de 20 à 70 % en poids, de préférence de 30 à 50 % en poids, sur la base du poids sec de l'amidon.

**12.** Procédé selon l'une quelconque des revendications précédentes, dans lequel l'amidon hydrophobe possède une teneur en amylopectine supérieure à 80 % en poids, de préférence supérieure à 95 % en poids, sur la base du poids sec de l'amidon.

**13.** Procédé selon l'une quelconque des revendications précédentes, dans lequel l'amidon hydrophobe est un amidon hydrophobe cuit.

**14.** Procédé selon l'une quelconque des revendications précédentes, dans lequel ledit produit alimentaire comprend un émulsifiant,

**15.** Procédé selon l'une quelconque des revendications 2 à 14, dans lequel au plus 15 % en poids dudit amidon cuit et/ou dudit amidon prégélatinisé sont remplacés par ledit amidon hydrophobe, de préférence au plus 10 % en poids, plus préférablement au plus 5 % en poids.

**16.** Procédé selon l'une quelconque des revendications 2 à 15, dans lequel ledit amidon cuit est un amidon de pomme de terre, de manioc, de blé, d'orge, de maïs, de riz, de pois ou de haricot.

**17.** Utilisation d'un amidon hydrophobe en tant qu'agent de réduction d'absorption d'huile pendant la friture, dans laquelle, l'amidon hydrophobe est obtenu en faisant réagir un amidon avec un agent hydrophobe comprenant une chaîne alkyle ou alcényle constituée de 4 à 24 atomes de carbone.

**EP 1 856 986 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0189935 A
- EP 0689829 A
- US 5776476 A
- WO 9424169 A